# EUROPEAN PATENT SPECIFICATION

(11) **EP 1 562 894 B1**
(45) Date of publication and mention of the grant of the patent: **24.01.2007**
(21) Application number: 03779857.6
(22) Date of filing: 03.11.2003
(51) Int. Cl.: C07C 253/30, C07C 255/42, C07C 255/24, C07C 255/44

(54) **PROCESS FOR THE RACEMISATION OF ENANTIOMERICALLY ENRICHED ALPHA-AMINO NITRILES**
VERFAHREN ZUR RACEMISIERUNG ENANTIOMERANGEREICHERTER ALPHA-AMINONITRILE
METHODE DE RACEMISATION D'ALPHA-AMINONITRILES ENRICHIS D'ENANTIOMERES

(30) Priority: 21.11.2002 EP 02102614
(43) Date of publication of application: 17.08.2005
(73) Proprietor: DSM IP Assets B.V., 6411 TE Heerlen (NL)
(72) Inventor: VERZIJL, Gerardus, Karel, Maria, NL-5855 AR Well (NL); BROXTERMAN, Quirinus, Bernardus, NL-6151 JA Munstergeleen (NL)
(74) Representative: Breepoel, Peter M.
(86) International application number: PCT/EP2003/012412
(87) International publication number: WO 2004/046088

(56) References cited:
- EP-A- 1 050 529
- US-A- 4 683 324
- LOPEZ-SERRANO P ET AL: "Enantioselective acylation of alpha-aminonitriles catalysed by Candida antarctica lipase. An unexpected turnover-related racemisation" TETRAHEDRON: ASYMMETRY, ELSEVIER SCIENCE PUBLISHERS, AMSTERDAM, NL, vol. 12, no. 2, 19 February 2001 (2001-02-19), pages 219-228, XP004230902 ISSN: 0957-4166
- DATABASE WPI Derwent Publications Ltd., London, GB; AN 1993-383032 XP002237430 "IMPROVED RACEMISATION FOR 2-AMINO:NITRILE(S) - COMPRISES CONTACTING OPTICALLY ACTIVE 2-AMINO:NITRILE(S) WITH POTASSIUM CYANIDE IN BENZENE-METHANOL MIXTURE" -& JP 05 286919 A ((NIHA) NIKKO KYOSEKI KK), 2 November 1993 (1993-11-02)
- IMELIK B. ET AL: 'Catalysis by Zeolites', 1980, ELSEVIER SCIENTIFIC PUBLISHING COMPANY, AMSTERDAM Y. Ono, Role of basic sites in catalysis by zeolites page 19, section 2.1
- ROLAND E.; KLEINSCHMIT P.: 'Ullmann's Encyclopedia of Industrial Chemistry', 2002, WILEY-VCH VERLAG GMBH & CO. Zeolites section 7.3, first paragraph

## Description

The invention relates to a process for the racemisation of enantiomerically enriched α-amino nitriles.

Many enantiomerically enriched active ingredients like pharmaceuticals and agrochemicals can be prepared using enantiomerically enriched α-amino nitriles, which are commonly used as a precursor for α-amino acids or derivatives thereof. The preparation of pharmaceuticals or agrochemicals generally involves a process with many consecutive steps. α-Amino nitriles can be converted into a wide variety of compounds, for example α-amino acids, α-amino acid amides, β-amino alcohols, vicinal diamines and protected α-amino aldehydes, and therefore are broadly applicable in the preparation of enantiomerically enriched active ingredients. The preparation of enantiomerically enriched compounds commonly includes a resolution step wherein the undesired enantiomer is left as a byproduct. In such resolutions the theoretical maximum yield with regard to the starting material is 50%. The yield can increase significantly by racemisation and recycling of the undesired enantiomer. Preferably the resolution process is combined with racemisation in situ. Therefore, there exists a need for a racemisation process of α-amino nitriles that is compatible with a large variety of resolution processes.

A process wherein an in situ racemisation of an enantiomerically enriched α-amino nitrile occurs is described in US-A-4683324.

A disadvantage of the process of US-A-4683324 is that a protic solvent has to be used. It is known that α-amino nitriles partly decompose in protic solvents. Therefore, in the process of US-A-4683324 HCN is added in order to suppress decomposition. From EP-A-1050529 it is further known that non polar, (but actually meaning aprotic) solvents stabilize α-amino nitriles with respect to decomposition and prevent racemisation.

It is the object of the invention to provide a process for the racemisation of α-amino nitriles wherein a racemisation with less decomposition is achieved, and that can be used in combination with resolutions in the preparation of enantiomerically enriched compounds in high yield and high enantiomeric excess.

This object is achieved according to the invention by using a Lewis acid catalyst. Lewis acids by definition are able to accept an electron pair from compounds that have a free available electron pair.

Suitable Lewis acid catalysts according to the invention comprise a metal ion M , which is complexed according to the genaral structure

MₙXₚS_{q}Lᵣ

in which n represents an integer larger than or equal to 1, for instance is equal to 1, 2, 3, 4, 5, 6, 7..... and p, q and r each independently represent an integer larger than or equal to 0, for instance 0, 1, 2, 3, 4, 5, 6, 7, ..., and in which n and p are chosen such that MₙXₚ is neutral.

The metal M may be in the ionic form (M^{k+}) with suitable counter ions X, wherein k represents the valence of the metal ion, or in the zero valent form (M⁰) with p = 0.

Suitable metals are for instance metals chosen from main group elements IA-IVA of the Periodic Table (CAS version), the transition metals and the lanthanides, preferably AI, Ti, Zr, or a lanthanide, for example Sm or La, or combinations thereof.

X represents an anionic counter ion or covalently bound anionic ligand for non zero valent metals. Suitable examples are halides, in particular Cl- or Br⁻; alkyl groups with e.g. 1-12 C-atoms, for example methyl, ethyl, n-propyl (ⁿPr) or i-butyl (ⁱBu) groups, alkoxy groups with e.g. 1-12 C-atoms, for example n-pentoxy, i-propoxy, t-butoxy groups preferably the alkoxy groups derived from a secondary alcohol; anions derived from amides, aminoalcohols or amines; CN⁻ groups; anionic aromatic ligands, in particular cyclopentadienyl (Cp), pentamethyl cyclopentadienyl (Cp*) or indenyl.

S represents a so-called spectator ligand, a neutral ligand which is difficult to exchange by other ligands, for example aromatic compounds or olefines. Examples of aromatic compounds are aromatic hydrocarbons, for instance benzene, toluene, cumene, cymene or mesitylene. Suitable olefines are for instance dienes, in particular 1,5-cyclooctadiene or 1,5-hexadiene.

L represents a neutral ligand, which can be relatively easy exchanged by other ligands, for example phosphines in particular PPh₃ or PCy₃, nitriles or coordinating solvent molecules, especially tetrahydrofuran (THF), acetonitrile, dimethylfomamide, alcohols, amines, in particular tertiary amines, for example Et₃N.

Catalyst represented by the general structure MₙXₚS_{q}Lᵣ as described above are generally known in the art. As will be understood by the person skilled in the art the distinction between spectator ligand S and neutral ligands L is gradual and amenable to discussion; besides that it may depent on the reaction conditions.

Examples of suitable catalyst are: LiBr, Al(OⁱPr)₃, AlCl₃, Al(alkyl)₃, with each alkyl is a C1-C12 alkyl group, Al(Et₂)CN, Al(ⁱBu)₂H, -Al(̵CH₃)-O)̵ₙ-, Zr(O^{t}Bu)₄, Zirconocene, Sm(OⁱPr)₃, Sm(Cp)₃, La(Cp)₃. Particularly preferred catalyst are aluminum alkoxides, aluminum alkyls, lanthanide alkoxides, for instance samarium alkoxides, and lanthanocenes, for instance lanthanide tris-cyclopentadienyl compounds.

The racemisation can be carried out in the presence of a solvent. Preferably aprotic solvents are used in the racemisation according to the invention, for instance aliphatic hydrocarbons, for example hexane, heptane; aromatic hydrocarbons, for example toluene and xylene; ethers, for example methyl t-butyl ether (MTBE); esters, for example ethyl acetate; or mixtures thereof. The concentration of the amino nitrile in the reaction mixture preferably is between 0.1 and 2 M.

The temperature for the racemisation preferably will be chosen as high as possible and such that still a sufficiently low level of decomposition is maintained under the reaction conditions. The optimal temperature can be determined by the skilled person and lies for instance between -20 and 120 °C, preferably between 30 and 100 °C.

The racemisation according to the invention can be combined with a separate resolution processes or with an in situ resolution process so that in principle the product can be obtained in 100% yield and 100% ee. In a particularly preferred embodiment of the invention, racemisation is applied in combination with an in situ resolution process.

Examples of resolution processes that can be combined with the racemisation according to the invention are for instance, crystallization induced resolutions (classical resolutions), for instance resolutions via diastereoisomeric salt formation or entrainment, enzymatic resolutions, or resolutions achieved by physical separation methods. Examples of resolution process in combination with an insitu racemisation are, for instance, crystallization induced asymmetric transformations or dynamic kinetic resolutions (DKR). A clear advantage of the racemisation according to the invention is that an aprotic solvent can be chosen. In order to achieve a "100% yield, 100° ee" result as a rule it is better to use aprotic solvents in resolution processes. For instance, aprotic organic solvents show less deactivation of enzymes in enzymatic processes than protic organic solvents. Thus, in combination with an enzymatic resolution (DKR) aprotic solvents are particularly suitable solvents to be used in stereoselective reactions e.g. stereoselective acylation reactions or stereoselective nitrile converting reactions. For asymmetric transformations low solubilities of diastereomeric salts are preferred and can be achieved by aprotic solvents.

In such "100% yield and 100% ee" concepts the racemisation system is chosen such that the racemisation system is compatible with the stereo differentiating process, for instance with the resolving agents or the enzyme used in the resolution. The choice of the optimum reaction conditions will as a rule be a compromise between the optimum resolving or stereo differentiating process conditions and the optimum racemisation conditions. The person skilled in the art will be able to find the optimal conditions for his situation. The temperature range, for instance, mostly will be chosen between 0 and 120 °C, preferably 20 and 110 °C. The concentration of the amino nitrile in the reaction mixture preferably is between 0.1 and 2M.

Examples of amino nitriles that can be racemised according to the invention are for instance amino nitriles with formula (1) 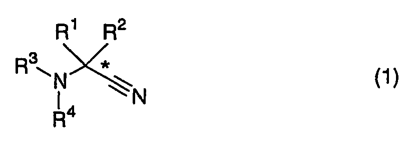 wherein * denotes a chiral C-atom, R¹, R², R³ and R⁴ each independently represent H, an optionally substituted alkyl, aryl, alkaryl or aralkyl group with, for instance 1-20 C-atoms, or R¹ and R² or R¹ and R³ or R ³ and R⁴ together with the atom(s) to which they are attached form an optionally substituted 4-8 membered (hetero)cyclic group with for instance 3-20 C-atoms.

The invention will be elucidated by the following examples, without however being limited thereby.

### Example I

### Racemisation using diethylaluminum cyanide

In a 5 ml vial, 0.1 mmol (R)-2-amino-3-phenylpropionitrile (e.e. > 99 %) was dissolved in 1 ml toluene. The vial was sealed with a teflon-lined cap and the reaction mixture was degassed by nitrogen. A 1 M solution of diethylaluminum cyanide in toluene was added by syringe and the reaction mixture was stirred under nitrogen at 80°C for 18 h. The results are given in Table 1.

**Table 1: Racemisation of (R)-2-amino-3-phenylpropionitrile**

| Exp. | Catalyst solution (µl) | Catalyst (mol %) | E.e. (%) |
|---|---|---|---|
| 1 | 10 | 10 | 0 |
| 2 | 25 | 25 | 0 |
| 3 | 50 | 50 | 0 |

### Example II

### Racemisations at room temperature using aluminum complexes

Fresh stock solutions (0.1 m in toluene) were prepared from a) diethylaluminum cyanide ((C₂H₅)₂AlCN)), b) triethylaluminum ((C₂H₅)₃Al), c) tri-i-butylaluminum [(CH₃)₂CHCH₂]₃Al and d) MAO (methylaluminoxane). each stock solution was used as catalyst solution in following racemisation procedure for 2-amino-3-phenylpropionitrile.

In a 5 ml vial, 0.1 mmol (R)-2-amino-3-phenylpropionitrile (e.e. > 99 %) was dissolved in 0.9 ml toluene. The vial was sealed with a teflon-lined cap and the reaction mixture was degassed by nitrogen. To the solution, 0.1 ml of catalyst solution was added by syringe and the reaction mixture was stirred under nitrogen at room temperature. The results are given in Table 2, below.

**Table 2: Racemisation of (R)-2-amino-3-phenylpropionitrile**

| Exp. | Catalyst | t (h) | E.e. (%) |
|---|---|---|---|
| 1 | C₂H₅)₂AlCN | 1 | 0 |
| 2 | ((C₂H₅)₃Al | 1 | 0 |
| 3 | [(CH₃)₂CHCH₂]₃Al | 0.25 | 14 |
| 4 | MAO | 17 | 43 |

The lowest e.e. represents the best result

### Example III

### Racemisation at room temperature using DIBAL

A 0.1 M stock solution of DIBAL (diisobutylaluminum hydride) in hexane was prepared by dilution from a 1 M solution in hexane.
In a 5 ml vial, 0.1 mmol (R)-2-amino-3-phenylpropionitrile (e.e. > 99 %) was dissolved in 0.9 ml toluene. The vial was sealed with a teflon-lined cap and the reaction mixture was degassed by nitrogen. To the solution, 0.1 ml of the 0.1 M stock solution in hexane was added by syringe and the reaction mixture was stirred under nitrogen at room temperature. After 1.25 h, 2-amino-3-phenylpropionitrile was racemised to 59 % e.e..

### Example IV

### Racemisation of (R)-2-amino-3-phenylpropionitrile using heterogeneous racemisation catalyst

Catalyst and solid (R)-2-amino-3-phenylpropionitrile (0.1 mmol, e.e. > 99 %) were weight under a dry atmosphere in a glovebox in a pre-dried 5 ml vial, equipped with magnetic stirrer bar. The vial was sealed with a teflon-lined cap and taken out of the glovebox. To the heterogeneous mixture, 1 ml dry solvent was added by syringe, and the reaction mixture was stirred under nitrogen at temperature given in Table 3 below.

**Table 3: Racemisation of (R)-2-amino-3-phenylpropionitrile using heterogeneous catalysts**

| Exp. | Catalyst | Catalyst (mg) | Solvent | T (°C) | t (h) | e.e. (%) |
|---|---|---|---|---|---|---|
| 1 | Sm₅O[OC₃H₇]₁₃ | 60 | THF | 75 | 17 | 10 |
| 2 | Sm₅O[OC₃H₇]₁₃ | 1.5 | THF | 75 | 24 | 92 |
| 3 | Sm₅O[OC₃H₇]₁₃ | 1.5 | Toluene | 75 | 24 | 70 |
| 4 | La(Cp)₃ | 33.4 | Toluene | 80 | 1 | 0 |
| 5 | La(Cp)₃ | 3.3 | Toluene | 50 | 17 | 7 |
| 6 | Sm(Cp)₃ | 3.5 | Toluene | 50 | 17 | 23 |
| 7 | Al₂O₃ | 10.3 | Toluene | 60 | 24 | 35 |
| 8 | SiO₂ | 24 | Toluene | 60 | 16 | 88 |

### Example V

### Racemisation of (R)-2-amino-3-phenylpropionitrile using aluminum isopropoxide

### A) Catalysis with solid aluminum isopropoxide

Solid aluminum isopropoxide (Al(O'Pr)₃) and solid (R)-2-amino-3-phenylpropionitrile (0.1 mmol, e.e. > 99 %) were placed in a vial of 5 ml and dissolved in 1 ml solvent. The vial was sealed with a teflon-lined cap, the solution degassed by nitrogen and the racemisation was carried out at temperatures given in Table 4 below.

**Table 4: Racemisation of (R)-2-amino-3-phenylpropionitrile using solid Al(OⁱPr)₃**

| Exp. | Catalyst (mg) | Solvent | T (°C) | t (h) | e.e. (%) |
|---|---|---|---|---|---|
| 1 | 2 | THF | 75 | 17 | 23 |
| 2 | 10 | THF | 75 | 17 | 0 |
| 3 | 0.8 | Toluene | 75 | 18 | 29 |
| 4 | 2 | Toluene | 20 | 70 | 83 |
| 5 | 3.4 | Toluene | 80 | 2 | 72 |

### B) Aluminum isopropoxide prepared from other aluminum derivatives

### Catalyst solution 1:

Aluminum isopropoxide derived from diethylaluminum cyanide (C₂H₅)₂AlCN) Into a 25ml vial was added isopropanol (3 mmol) and toluene (9 ml). The vial was sealed with a teflon-lined cap and the solution was degassed by nitrogen. To the solution, 1 ml of a 1 M solution of (C₂H₅)₂AlCN in toluene was added under nitrogen atmosphere.

### Catalyst solution 2:

Aluminum isopropoxide derived from diethylaluminum cyanide (C₂H₅)₂AlCN) Similar procedure as for catalyst solution 1 followed by heating at 80°C for 1 h.

### Catalyst solution 3:

Aluminum isopropoxide derived from triethylaluminum ((C₂H₅)₃Al).
Into a 25-ml vial was added isopropanol (4 mmol) and toluene (9 ml). The vial was sealed with a teflon-lined cap and the solution was degassed by nitrogen. To the solution, 1 ml of a 1 M solution of (C₂H₅)₃Al in toluene was added under nitrogen atmosphere.
The obtained catalyst solution was allowed to stand for 1 night at room temperature.

Catalyst solutions 1, 2 and 3 were used in standard racemisation protocol for (R)-2-amino-3-phenylpropionitrile:
In a 5 ml vial, 0.1 mmol (R)-2-amino-3-phenylpropionitrile (e.e. > 99 %) was dissolved in 0.9 ml toluene. The vial was sealed with a teflon-lined cap and the reaction mixture was degassed by nitrogen. To the solution, catalyst solution was added by syringe and the reaction mixture was heated at temperature given in Table 5 below.

**Table 5: Racemisation of (R)-2-amino-3-phenylpropionitrile using Al(O'Pr)₃ derived from aluminum derivatives**

| Exp. | Cat. sol | Catalyst (ml) | T (°C) | t (h) | e.e. (%) |
|---|---|---|---|---|---|
| 1 | 1 | 0.1 | 20 | 21 | 20 |
| 2 | 2 | 0.1 | 60 | 1 | 15 |
| 3 | 3 | 0.1 | 50 | 4 | 9 |
| 4 | 3 | 0.05 | 60 | 1 | 56 |
| 5 | 3 | 0.1 | 60 | 1 | 32 |

### C) Aluminum isopropoxide prepared from other aluminum derivatives, catalytic racemisation in the presence of triethylamine

### Catalyst solution:

Aluminum isopropoxide derived from trimethylaluminum ((CH₃)₃Al).
Into a 25-ml vial was added isopropanol (3 mmol) and toluene (9.5 ml). The vial was sealed with a teflon-lined cap and the solution was degassed by nitrogen. To the solution, 0.5 ml of a 2 M solution of (CH₃)₃Al in toluene is added under nitrogen atmosphere.
The obtained catalyst solution was allowed to stand for 15 minutes at room temperature and for 15 minutes at 80°C respectively.
Stock solution triethylamine:
Into a 50-ml vial was added triethylamine (2,54 mmol) and toluene (25 ml). The vial was sealed with a teflon-lined cap and stored at room temperature.
Catalyst solution was used in standard racemisation protocol for (R)-2-amino-3-phenylpropionitrile in presence of triethylamine:
In a 5 ml vial, 0.1 mmol (R)-2-amino-3-phenylpropionitrile (e.e. > 99 %) was dissolved in toluene and stock solution of triethylamine was added. The vial was sealed with a teflon-lined cap and the reaction mixture was degassed by nitrogen. To the solution, catalyst solution was added by syringe and the reaction mixture was heated at 60°C for 19 h.

**Table 6: Racemisation of (R)-2-amino-3-phenylpropionitrile using Al(OⁱPr)₃ derived from (CH₃)₃Al in the presence of triethylamine**

| Exp. | Toluene (ml) | Catalyst solution (ml) | Stock triethylamine(ml) | e.e. (%) |
|---|---|---|---|---|
| 1 | 0.9 | 0.1 | 0 | 8 |
| 2 | 0.9 | 0 | 0.1 | > 99 |
| 3 | 0.8 | 0.1 | 0.1 | 27 |

### D) Racemisation of (R)-2-amino-3-phenylpropionitrile using Al(OⁱPr)₃ prepared from trimethylaluminum ((CH₃)₃Al) v.s. solid Al(OⁱPr)₃

Catalyst solution was derived from trimethylaluminum ((CH₃)₃Al). Into a 25-ml vial was added isopropanol (3 mmol) and toluene (9.5 ml). The vial was sealed with a teflon-lined cap and the solution was degassed by nitrogen. To the solution, 0.5 ml of a 2 M solution of (CH₃)₃Al in toluene is added under nitrogen atmosphere.

The obtained catalyst solution was allowed to stand for 15 minutes at room temperature and for 15 minutes at 80°C, respectively.
In a 5 ml vial, 0.1 mmol (R)-2-amino-3-phenylpropionitrile (e.e. > 99 %) and solid Al(OⁱPr)₃ was dissolved in toluene. The vial was sealed with a teflon-lined cap and the reaction mixture was degassed by nitrogen. To the solution, catalyst solution was added by syringe and the reaction mixture was heated at 60°C for 2 h. The results are given in Table 7.

**Table 7: Racemisation of (R)-2-amino-3-phenylpropionitrile using Al(OⁱPr)₃**

| Exp. | Toluene (ml) | Catalyst solution (ml) | Al(O*ⁱ*Pr)₃ (mg) | e.e. (%) |
|---|---|---|---|---|
| 1 | 1 | 0 | 3.4 | 72 |
| 2 | 0.9 | 0.1 | 0 | 16 |

### Example VI

### Racemisation of (R)-2-amino-3-phenylpropionitrile using Al(OⁱPr)₃ in the presence of trimethylsilyl cyanide (TMS-CN)

In a 5 ml vial, toluene (1 ml) was added to 0.1 mmol (R)-2-amino-3-phenylpropionitrile (e.e. > 99 %), Al(O'Pr)₃ (0.01 mmol) and TMS-CN. The vial was sealed with a teflon-lined cap and the reaction mixture was degassed by nitrogen. The temperature of the reaction mixture was increased to 80°C for 3 h. The results are given in Table 8.

**Table 8: Racemisation of (R)-2-amino-3-phenylpropionitrile using Al(O'Pr)₃ in the presence of TMS-CN**

| Exp. | TMS-CN (mmol) | e.e. (%) |
|---|---|---|
| 1 | 0.1 | 23 |
| 2 | 0 | 56 |

### Example VII

### Racemisation of (R)-2-amino-3-phenylpropionitrile using diethylaluminum cyanide (C₂H₅)₂AlCN) in combination with isopropanol

### Catalyst solution:

Into a 50-ml vial was added toluene (10 ml). The vial was sealed with a teflon-lined cap and the toluene was degassed by nitrogen. To the toluene is added under nitrogen atmosphere respectively, 0.4 ml of a 1 M solution of (C₂H₅)₂AlCN in toluene and isopropanol (8.5 mmol).
The obtained catalyst solution was allowed to stand for 1 night at room temperature and used in catalytic racemisation of (R)-2-amino-3-phenylpropionitrile:
In a 5 ml vial, 0.1 mmol (R)-2-amino-3-phenylpropionitrile (e.e. > 99 %) was dissolved in 1 ml toluene. The vial was sealed with a teflon-lined cap and the reaction mixture was degassed by nitrogen. To the solution, catalyst solution was added by syringe and the temperature was increased to 80°C. The results are given in Table 9.

**Table 9: Racemisation of (R)-2-amino-3-phenylpropionitrile using (C₂H₅)₂AlCN/isopropanol (2eq.)**

| Exp. | Catalyst solution (µl) | Catalyst (mol %) | t (h) | e.e. (%) |
|---|---|---|---|---|
| 1 | 50 | 2 | 6 | 37 |
| 2 | 250 | 10 | 1 | 13 |

### Example VIII

### Racemisation of (R)-2-amino-3-phenylpropionitrile using aluminum/amine catalyst

### Catalyst solution 1:

Into a 50-ml vial was added benzylamine (2 mmol) and toluene (9 ml). The vial was sealed with a teflon-lined cap and the solution was degassed by nitrogen. To the solution, 1 ml of a 1 M solution of (C₂H₅)₂AlCN in toluene was added under nitrogen atmosphere.

### Catalyst solution 2, 3 and 4:

Into a 25-ml vial was added amine (3 mmol) and toluene (9 ml). The vial was sealed with a teflon-lined cap and the solution was degassed by nitrogen. To the solution, 0.5 ml of a 2 M solution of (CH₃)₃Al in toluene was added under nitrogen atmosphere. Temperature was increased to 80°C for 1 night.

### Catalyst solutions

| Catalyst solution | Amine |
|---|---|
| 2 | (S)-1-phenylethylamine |
| 3 | (RS)-1-phenylethylamine |
| 4 | N-methylbenzylamine |

Catalyst solutions were used in the catalytic racemisation of (R)-2-amino-3-phenylpropionitrile:
In a 5 ml vial, 0.1 mmol (R)-2-amino-3-phenylpropionitrile (e.e. > 99 %) was dissolved in 0.9 ml toluene. The vial was sealed with a teflon-lined cap and the reaction mixture was degassed by nitrogen. To the solution, 0.1 ml catalyst solution was added by syringe and the temperature was increased to 60°C for 1 hour. The results are given in Table 10.

**Table 10: Racemisation of (R)-2-amino-3-phenylpropionitrile using aluminum/amine catalyst**

| Catalyst solution | e.e. (%) |
|---|---|
| 1 | 27 |
| 2 | 13 |
| 3 | 34 |
| 4 | 10 |

### Example IX

### Racemisation of (R)-2-amino-3-phenylpropionitrile using diethylaluminum cyanide (C₂H₅)₂AlCN in presence of isopropyl acetate

### Catalyst solution:

Into a 50-ml vial was added toluene (9 ml). The vial was sealed with a teflon-lined cap and the solution was degassed by nitrogen. To the solution, 1 ml of a 1 M solution of (C₂H₅)₂AlCN in toluene was added under nitrogen atmosphere.
Catalyst solution was used in standard racemisation protocol for (R)-2-amino-3-phenylpropionitrile in presence of isopropyl acetate:
In a 5 ml vial, 0.1 mmol (R)-2-amino-3-phenylpropionitrile (e.e. > 99 %) and isopropyl acetate (0.11 mmol) were dissolved in 0,9 ml toluene. The vial was sealed with a teflon-lined cap and the reaction mixture was degassed by nitrogen. To the degassed solution, 0.1 ml catalyst solution was added by syringe and the reaction mixture was allowed to stand at ambient temperature for 1 h, after which 2-amino-3-phenylpropionitrile was racemised to 23 % e.e..

### Example X

### Racemisation of (R)-2-amino-3-phenylpropionitrile using lithium salts

LiBr was dried under vacuum at 300°C prior to use.
New LiPF₆ was used as such in glovebox.

### Stock lithium salts

In a glovebox, lithium salt was added to a 25 ml-vial. The vial was sealed with a teflon-lined cap and taken out of the glovebox. Dry THF was added and the lithium salt was dissolved.

| Catalyst solution | Lithium salt (mmol) | THF (ml) |
|---|---|---|
| LiBr | 1,5 | 15 |
| LiPF₆ | 1 | 10 |

Racemisation of (R)-2-amino-3-phenylpropionitrile using lithium salts
To a 5 ml vial, 0.1 mmol (R)-2-amino-3-phenylpropionitrile (e.e. > 99 %) was added.
The vial was sealed with a teflon-lined cap and degassed by nitrogen. To the (R)-2-amino-3-phenylpropionitrile-containing vial was added catalyst solution (1 ml) and the 2-amino-3-phenylpropionitrile was dissolved. The racemisation was performed under nitrogen at given temperature. The results are summarized in Table 11.

**Table 11: Catalytic racemisation using lithium salts**

| Exp. | Catalyst solution | T (°C) | t (h) | E.e. (%) |
|---|---|---|---|---|
| 1 | LiBr | 80 | 24 | 34 |
| 2 | LiPF₆ | 60 | 2 | 40 |

### Example XI

### Racemisation of (R)-2-amino-3-phenylpropionitrile nitrile using early transition metal catalysts

Racemisation using Zr(OBuⁱ)4

### Preparation catalyst solution, a general procedure:

Zr(OBu*^{t}*)₄ was placed in a 5 ml vial under an atmosphere of dry nitrogen and toluene, freshly distilled from sodium metal, was introduced and the vial was sealed with a teflon-lined cap.

**Table Zr(OBu^{t})₄ solutions in toluene**

| Solution (M) | Zr(OBu*^{t}*)₁ (mmol) | Toluene (ml) |
|---|---|---|
| 0.1 | 1 | 10 |
| 1 | 2 | 2 |

### Racemisation of (R)-2-amino-3-phenylpropionitrile

In a 5 ml vial, 0.1 mmol (R)-2-amino-3-phenylpropionitrile (e.e. > 99 %) was dissolved in 0.9 ml dry toluene. The vial was sealed with a teflon-lined cap and the reaction mixture was degassed by nitrogen. To this solution was added a toluene solution of Zr(OBu')₄ (0.1 ml) by syringe and the reaction mixture was stirred for 24 h at 80°C. The results are given in Table 12.

**Table 12: Catalytic racemisation of (R)-2-amino-3-phenylpropionitrile with Zr(OBu^{t})₄**

| Exp. | Catalyst solution (M) | E.e. (%) |
|---|---|---|
| 1 | 0.1 | 73 |
| 2 | 1 | 0 |

### Racemisation using different complexes

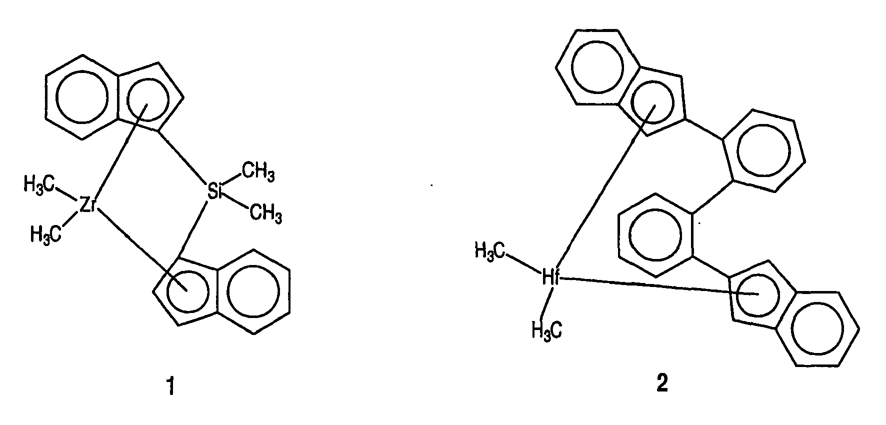

### Catalyst solution

The complex (0.1 mmol) was placed in a 5 ml vial under an atmosphere of dry nitrogen and toluene (1 ml) freshly distilled from sodium metal was introduced. The vial was sealed with a teflon-lined cap and the mixture was stirred until the complex was completely dissolved.

### Racemisation of (R)-2-amino-3-phenylpropionitrile.

In a 5 ml vial, 0.1 mmol (R)-2-amino-3-phenylpropionitrile (e.e. > 99 %) was dissolved in 0.9 ml dry toluene. The vial was sealed with a teflon-lined cap and the reaction mixture was degassed by nitrogen. To this solution was added catalyst solution (0.1 ml) by syringe and the reaction mixture was stirred for 24 h at 50°C. The results are given in Table 13.

**Table 13: Catalytic racemisation of (R)-2-amino-3-phenylpropionitrile**

| Exp. | Complex | E.e. (%) |
|---|---|---|
| 1 | 1 | 6 |
| 2 | 2 | 75 |

### Example XII

### Racemisation of (R)-2-amino-3-phenylpropionitrile using aluminum alkoxides

Aluminum alkoxide catalysts prepared from Me₃Al.
Preparation catalyst solution, a general procedure:
Alcohol (3 mmol) was placed in a 25 mi-vial and toluene (9.5 ml) freshly distilled from sodium metal was introduced. The vial was sealed with a teflon-lined cap and degassed by nitrogen. To this solution was added a 2 M toluene solution of Me₃Al (0.5 ml, 1 mmol) and a stream of dry nitrogen removed the generated methane. After standing for 15 minutes at room temperature, the temperature is increased to 80°C for a period of time given in Table.

### Racemisation of (R)-2-amino-3-phenylpropionitrile

In a 5 ml vial, 0.1 mmol (R)-2-amino-3-phenylpropionitrile (e.e. > 99 %) was dissolved in 0.9 ml dry toluene. The vial was sealed with a teflon-lined cap and the reaction mixture was degassed by nitrogen. To this solution was added catalyst solution (0.1 ml) by syringe and the temperature of the reaction mixture was increased to 60°C. Degrees of racemisation during a period of time are given in Table 14.

**Table 14: Racemisation of (R)-2-amino-3-phenylpropionitrile catalysed by aluminum alkoxides**

| | Preparation catalyst solution | | racemisation | |
|---|---|---|---|---|
| Exp. | Alcohol | t (h) at 80°C | t (h) | e.e. (%) |
| 1 | Isopropanol | 0.25 | 1 | 34 |
| 2 | (RS)-1-phenylethanol | 0.25 | 1 | 77 |
| 3 | (S)-1-phenylethanol^{a)} | 0.5 | 1 | 72 |
| 4 | 1-pentanol | 1 | 21 | 59 |
| 5 | 2-octanol | 0.5 | 1 | 44 |
| 6 | 2-cyano-2-propanol | 0.25 | 1.25 | 74 |
| 7 | (RS)-1-cyclohexylethanol | 1 | 1 | 55 |
| 8 | (RS)-1-(4-Chlorophenyl)ethanol | 1 | 1 | 56 |
| 9 | (RS)-1-(4-Bromophenyl)ethanol | 1 | 1 | 63 |

| | | | | |
|---|---|---|---|---|
| a) During catalyst preparation, e.e. of (S)1-phenylethanol dropped to 54 % | | | | |

### Aluminum alkoxide catalyst prepared from ⁱBu₃Al

Isopropanol (3 mmol) was placed in a 25 ml-vial and toluene (9 ml) freshly distilled from sodium metal was introduced. The vial was sealed with a teflon-lined cap and degassed by nitrogen. To this solution was added a 1 M toluene solution of *ⁱ*Bu₃Al (1 ml, 1 mmol) and a stream of dry nitrogen removed the generated butane. After standing for 15 minutes at room temperature, the temperature is increased to 80°C for a period of time given in Table.

### Racemisation of (R)-2-amino-3-phenylpropionitrile.

In a 5 ml vial, 0.1 mmol (R)-2-amino-3-phenylpropionitrile (e.e. > 99 %) was dissolved in 0.9 ml dry toluene. The vial was sealed with a teflon-lined cap and the reaction mixture was degassed by nitrogen. To this solution was added catalyst solution (0.1 ml) by syringe and the temperature of the reaction mixture was increased to 60°C. Degree of racemisation is given in Table 15.

**Table 15: Racemisation of (R)-2-amino-3-phenylpropionitrile catalysed by IPA/ⁱBu₃Al**

| | Preparation catalyst solution | | racemisation | |
|---|---|---|---|---|
| Exp. | Alcohol | t (h) at 80°C | t (h) | e.e. (%) |
| 1 | Isopropanol | 0.25 | 1 | 61 |
| 2 | Isopropanol | 1.25 | 1 | 42 |

### Example XIII

### Racemisation of (R)-2-amino-3-phenylpropionitrile catalysed by bis(1.5-cyclooctadiene)rhodium (I) tetrafluoroborate

(R)-2-Amino-3-phenylpropionitrile (e.e. > 99 %) (146 mg, 0.1 mmol) and bis(1,5-cyclooctadiene)rhodium (I) tetrafluoroborate (4.5 mg, 0.011 mmol) were placed in a dry 5 ml vial. The vial was sealed with a teflon-lined cap, freshly distilled toluene (0.9 ml) was introduced and the reaction mixture was degassed by nitrogen. The temperature of the reaction mixture was increased to 60°C for 18 h, after which time (R)-2-Amino-3-phenylpropionitrile was racemised to 62 % e.e..

### Example XIV

### Racemisation of (R)-2-amino-3-phenylpropionitrile using metal isopropoxides

### Catalyst solution

Into a previously dried 25-ml vial was added metal isopropoxide (1 mmol) under an atmosphere of dry nitrogen. To the metal isopropoxide was added freshly from sodium distilled toluene (10 ml) and the vial was sealed with a teflon-lined cap.

### Racemisation of (R)-2-amino-3-phenylpropionitrile

A 5 ml vial was charged with 0.1 mmol (R)-2-amino-3-phenylpropionitrile (e.e. > 99 %) and dissolved in 0.9 ml dry toluene. The vial was sealed with a teflon-lined cap and the homogeneous solution was degassed by nitrogen. To this solution was added catalyst solution (0.1 ml) by syringe and the temperature of the reaction mixture was increased to 60°C. Degrees of racemisation during a period of time using different metal catalysts are given in Table 16.

**Table 16: Racemisation of (R)-2-amino-3-phenylpropionitrile catalysed by M(OⁱPr)₃**

| Exp. | M(O*ⁱ*Pr)₃ | racemisation | |
|---|---|---|---|
| | | t (h) | e.e. (%) |
| 1 | Ga(OPr)₃ | 1 | 85 |
| 2 | Sc(O'Pr)₃ | 5 | 92 |
| | | 21 | 77 |
| 3 | Y(O'Pr)₃ | 5 | 46 |
| | | 21 | 23 |
| 4 | Yb(O*ⁱ*Pr)₃ | 24 | 54 |

### Example XV

### Racemisation of (R)-2-amino-2,3-dimethylbutyronitrile using Al(OⁱPr)₃

In two 5 ml vial, 0.1 mmol (*R*)-2-amino-2,3-dimethylbutyronitrile (e.e. 86 %) was weighed. To these vails 0.9 ml of toluene was added. The vials were sealed with teflon-lined caps and the solutions were degassed by nitrogen. To one of the degassed solution, 0.2 ml of a 0.1 M Al(OⁱPr)₃ catalyst solution in toluene was added by syringe. The temperature of the reaction mixture and blank reaction was increased to 60°C.
After ½ hour a sample of the reaction mixture and blank was taken and analysed by HPLC. At this point the reaction mixture was racemic. The results are given in Table 17.

**Table 17: Racemisation of (R)-2-amino-2,3-dimethylbutyronitrile using Al(O'Pr)₃**

| Exp. | Catalyst solution (ml) | t (h) | e.e. (%) |
|---|---|---|---|
| 1 | 0 | 0.5 | 86 |
| 2 | 0.2 | 0.5 | <5 |

### Example XVI

### Racemisation of (R)-2-amino-2.3-dimethylbutyronitrile using AlEt₃

In two 5 ml vial, 0.1 mmol (R)-2-amino-2,3-dimethylbutyronitrile (e.e. 86 %) was weighed. To these vails 0.9 ml of toluene was added. The vials were sealed with teflon-lined caps and the reaction mixture and blank were degassed by nitrogen. To one of the degassed solutions, 0.2 ml of a 0.1 M AlEt₃ catalyst solution in toluene was added by syringe. The reaction mixture and blank reaction were allowed to stand at room temperature. After 5 minutes a sample of the reaction mixture and blank was taken and analysed by HPLC. At this point the reaction mixture was racemic. The results are given in Table 18.

**Table 18: Racemisation of (R)-2-amino-2,3-dimethylbutyronitrile using AlEt₃**

| Exp. | Catalyst solution (ml) | t (min) | e.e. (%) |
|---|---|---|---|
| 1 | 0 | 5 | 86 |
| 2 | 0.2 | 5 | <5 |

### Example XVII

### Racemisation of (R)-2-amino-2,3-dimethylbutyronitrile using Zr(^{t}BuO)₄

In two 5 ml vials, 0.1 mmol (R)-2-amino-2,3-dimethylbutyronitrile (e.e. 86 %) was weighed. To these vails 0.4 ml of toluene was added. The vials were sealed with teflon-lined caps and the reaction mixtures were degassed by nitrogen. To one of the degassed solutions, 0.6 ml of a 1 M Zr(^{t}BuO)₄ catalyst solution in toluene was added by syringe. The temperature of the reaction mixture and blank reaction was increased to 80 °C. After 1 day a sample of the reaction mixture and blank was taken and analysed by HPLC. At this point the reaction mixture was racemic. The results are given in Table 19.

**Table 19: Racemisation of (R)-2-amino-2,3-dimethylbutyronitrile using Zr(^{t}BuO)₄**

| Exp. | Catalyst solution (ml) | t (h) | e.e. (%) |
|---|---|---|---|
| 1 | 0 | 24 | 86 |
| 2 | 0.6 | 24 | <5 |

### Example XVIII

### Racemisation of (R)-2-amino-3-methylbutyronitrile using Al(OⁱPr)₃

In two 5 ml vial, 0.1 mmol (R)-2-amino-3-methylbutyronitrile (e.e. 74 %) was weighed.

To both 0.9 ml toluene was added. The vials were sealed with a teflon-lined cap and the reaction mixtures were degassed by nitrogen. To one of the degassed solution, 0.1 ml of a 0.1 M Al(O'Pr)₃ catalyst solution in toluene was added by syringe. The temperature of the reaction mixtures was increased to 60°C. After 55 minutes a sample of the reaction mixture and the blank was taken and analysed by HPLC. At this point the reaction mixture was racemic. The results are given in Table 20.

**Table 20: Racemisation of (R)-2-amino-3-methylbutyronitrile using Al(O'Pr)₃**

| Exp. | Catalyst solution (ml) | t (min) | e.e. (%) |
|---|---|---|---|
| 1 | 0 | 55 | 74 |
| 2 | 0.1 | 55 | <5 |

### Example XIX

### Dynamic Kinetic Resolution of (R)-2-amino-3-phenylpropionitrile by racemisation with Al(OⁱPr)₃ and enzymatic acylation catalyzed by Novozyme 435®

To a solution of 75 mg (0.51 mmol) (R)-2-amino-3-phenylpropionitrile and 104 mg (0.59 mmol) i-propyl phenylacetate in 4.2 g o-xylene was added 201 mg of Novozyme 435®. After stirring the reaction mixture for 20 minutes at ambient temperature, under reduced pressure (~100 mbar), 0.44 g (0.05 mmol) of a 0.1 M Al(O'Pr)₃ solution in toluene was added. The reaction mixture was stirred at 70ºC, under reduced pressure (~100 mbar) for 43 hours. After -20 hours another 185 mg Novozyme 435® and 0.42 g Al(O'Pr)₃ solution in toluene was added. The reaction was analysed by HPLC. After 43 hours N-phenylacetyl-(S)-2-amino-3-phenylpropionitrile was formed 64% e.e.(S). The starting material was partially racemised.

### Example XX

### Asymmetric transformation of 2-amino-3-phenylpropionitrile using Al(O'Pr)₃ as racemisation catalyst

The 1:1 diastereomeric salt of racemic 2-amino-3-phenylpropionitrile and N-(4-chlorobenzoyl)-L-glutamic acid was made by dissolving an 2.5 g (17 mmol) of 2-amino-3-phenylpropionitrile and 4.8 g (17 mmol) of N-(4-chlorobenzoyl)-L-glutamic acid in 68 ml of methanol. The mixture was stirred at room temperature till everything was dissolved. The methanol was evaporated under reduced pressure to yield 7.2 g of the 1:1 diastereomeric salt (HPLC analysis: 2-amino-3-phenylpropionitrile < 5% e.e.). In a Schlenk tube 0.860 g (2 mmol) of the 2-amino-3-phenylpropionitrile N-(4-chlorobenzoyl)-L-glutamic acid salt was dissolved in 4.8 ml toluene. This mixture was stirred and heated to 80°C overnight. Then, after cooling 0.2 ml of a 1 M Al(O'Pr)₃ solution in toluene was added. The reaction mixture was stirred and heated to 80°C for another 2 days. After cooling to room temperature the precipitate was filtered and washed with toluene. The residue (0.651 g) was analysed by HPLC. The results are given in Table 21. (Optical yield = yield x e.e.).

**Table 21: Asymmetric transformation of 2-amino-3-phenylpropionitrile with Al(OⁱPr)₃**

| Sample | Yield (%) | e.e. (%) | Optical yield (%) |
|---|---|---|---|
| Residue | 76 | 92 (R) | 70 |

### Example XXI

### Asymmetric transformation of 2-amino-3-phenylpropionitrile in toluene using Sm₅O(OⁱPr)₁₃ as racemisation catalyst

In 4.0 ml of methanol is dissolved 250 mg (1.7 mmol) of 2-amino-3-phenylpropionitrile, 480 mg (1.7 mmol) of N-(4-chlorobenzoyl)-L-glutamic acid and 100 mg of samarium iso-propoxide ( Sm₅O(OⁱPr)₁₃ ). The methanol was removed under reduced pressure, the residue was suspended in 2 ml of toluene. The toluene was evaporated to remove the last traces of methanol and to the residue was added 4 ml of toluene. The reaction mixture was stirred for 21.5 h at 80°C. After cooling the diastereomeric salt and the Sm₅O(OⁱPr)₁₃ was filtered off. This residue was suspended in CH₂Cl₂ and washed with saturated NaHCO₃ solution. The CH₂Cl₂ solution was dried and evaporated. The remaining (R)-2-amino-3-phenylpropionitrile (200 mg) was analysed by HPLC. The results are given in Table 22.

**Table 22: Asymmetric transformation of 2-amino-3-phenylpropionitrile (Phe-CN) with Sm₅O(OⁱPr)₁₃ in toluene**

| Sample | Yield (%) | e.e. (%) | Optical yield (%) |
|---|---|---|---|
| Phe-CN | 73 | 90 (R) | 66 |

### Example XXII

### Asymmetric transformation of 2-amino-3-phenylpropionitrile in heptane using Sm₅O(OⁱPr)₁₃ as racemisation catalyst

In 4.0 ml of methanol is dissolved 250 mg (1.7 mmol) of 2-amino-3-phenylpropionitrile, 480 mg (1.7 mmol) of N-(4-chlorobenzoyl)-L-glutamic acid and 100 mg of samarium iso-propoxide (Sm₅O(O'Pr)₁₃). The methanol was removed under reduced pressure, the residue was suspended in 2 ml of heptane. The heptane was evaporated to remove the last traces of methanol and to the residue was added 4 ml of heptane. The reaction mixture was stirred for 21.5 h at 80°C. After cooling the diastereomeric salt and the Sm₅O(OⁱPr)₁₃ was filtered off. This residue was suspended in CH₂Cl₂ and washed with saturated NaHCO₃ solution. The CH₂Cl₂ solution was dried and evaporated. The remaining (R)-2-amino-3-phenylpropionitrile (205 mg) was analysed by HPLC. The results are given in Table 23.

**Table 23: Asymmetric transformation of 2-amino-3-phenylpropionitrile (Phe-CN) with Sm₅O(OⁱPr)₁₃ in heptane**

| Sample | Yield (%) | e.e. (%) | Optical yield (%) |
|---|---|---|---|
| Phe-CN | 82 | 81 (R) | 66 |

## Claims

1. Process for the racemisation of an enantiomerically enriched α-amino nitrile **characterized in that** the enantiomerically enriched α-amino nitrile is contacted with a Lewis acid catalyst in an aprotic solvent.

2. Process according to claim 1, wherein the Lewis acid catalyst comprises a metal chosen from main group elements IA-IVA of the Periodic Table (CAS version), the transition metals and the lanthanides.

3. Process according to claim 2 wherein the metal is chosen from the group consisting of Al, Ti, Zr, or lanthanides.

4. Process according to any one of claims 1-3, wherein a catalyst with the general structure MₙXₚS_{q}Lᵣ is used, wherein M represents the metal, X represents an anionic counterion or covalently bound anionic ligand for non zero valent metals, S represents a spectator ligand, L represents a neutral ligand, n represents an integer larger than or equal to 1 and p, q and r each independently represent an integer larger than or equal to 0, and in which n and p are chosen such that MₙXₚ is neutral.

5. Process according to claim 4, wherein the catalyst is chosen from the group of aluminum alkoxides, aluminum alkyls, lanthanide alkoxydes and lanthanocenes.

6. Process according to any one of claims 1-5, wherein the racemisation is performed in combination with a resolution process.

7. Process according to claim 6, wherein the racemisation is performed in combination with an enzymatic resolution process.

8. Process according to claim 6, wherein the racemisation is performed in combination with a crystallization induced resolution.

9. Process according to any one of claims 6-8, wherein the resolution process is combined with racemisation in situ.

10. Process according to claim 9, wherein the racemisation is performed in situ in a crystallization induced asymmetric transformation process.

## Patentansprüche

1. Verfahren zur Racemisierung eines enantiomerangereicherten α-Aminonitrils, **dadurch gekennzeichnet, daß** das enantiomerangereicherte α-Aminonitril mit einem Lewissäurekatalysator in einem aprotischen Lösungsmittel in Kontakt gebracht wird.

2. Verfahren nach Anspruch 1, wobei der Lewissäurekatalysator ein aus den Hauptgruppenelementen IA-IVA der Periodentafel (CAS-Version), den Übergangsmetallen sowie den Lanthaniden gewähltes Metall umfaßt.

3. Verfahren nach Anspruch 2, wobei das Metall aus der Gruppe Al, Ti, Zr oder den Lanthaniden gewählt ist.

4. Verfahren nach einem der Ansprüche 1-3, wobei ein Katalysator mit der allgemeinen Struktur MₙXₚS_{q}Lᵣ verwendet wird, wobei M für das Metall, X für ein anionisches Gegenion oder einen kovalent gebundenen anionischen Liganden für nicht nullwertige Metalle, S für einen Zuschauerliganden, L für einen neutralen Liganden, n für eine ganze Zahl größergleich 1 steht und p, q und r jeweils unabhängig für eine ganze Zahl größergleich 0 stehen und wobei n und p so gewählt werden, daß MₙXₚ neutral ist.

5. Verfahren nach Anspruch 4, wobei der Katalysator aus der Gruppe der Aluminiumalkoholate, Aluminiumalkyle, Lanthanidalkoholate und Lanthanocene gewählt ist.

6. Verfahren nach einem der Ansprüche 1-5, wobei die Racemisierung in Kombination mit einem Trennungsverfahren durchgeführt wird.

7. Verfahren nach Anspruch 6, wobei die Racemisierung in Kombination mit einem enzymatischen Trennungsverfahren durchgeführt wird.

8. Verfahren nach Anspruch 6, wobei die Racemisierung in Kombination mit einer durch Kristallisation induzierten Trennung durchgeführt wird.

9. Verfahren nach einem der Ansprüche 6-8, wobei das Trennungsverfahren mit der Racemisierung in situ kombiniert wird.

10. Verfahren nach Anspruch 9, wobei die Racemisierung in situ in einem durch Kristallisation induzierten asymmetrischen Transformationsverfahren durchgeführt wird.

## Revendications

1. Processus de racémisation d'un α-amino nitrile enrichi en énantiomères, **caractérisé en ce que** le α-amino nitrile enrichi en énantiomères est mis en contact avec un catalyseur acide de Lewis dans un solvant aprotique.

2. Processus selon la revendication 1, dans lequel le catalyseur acide de Lewis comprend un métal choisi parmi des éléments d'un groupe principal IA-IVA de la table périodique (version CAS), les métaux de transition et les lanthanides.

3. Processus selon la revendication 2, dans lequel le métal est choisi dans le groupe composé de l'Al, du Ti, du Zr ou des lanthanides.

4. Processus selon l'une quelconque des revendications 1 à 3, dans lequel on utilise un catalyseur ayant la structure générale MₙXₚS_{q}Lᵣ, dans laquelle: M représente le métal; X représente un contre-ion anionique ou un ligand anionique, lié par covalence, pour des métaux de valence non nulle ; S représente un ligand spectateur ; L représente un ligand neutre ; n représente un entier supérieur ou égal à 1 ; et p, q et r représentent chacun, indépendamment, un entier supérieur ou égal à 0 ; et dans laquelle n et p sont choisis de sorte que MₙXₚ soit neutre.

5. Processus selon la revendication 4, dans lequel le catalyseur est choisi dans le groupe des alcoxydes d'aluminium, des alkyles d'aluminium, des alcoxydes de lanthanides et des lanthanocènes.

6. Processus selon l'une quelconque des revendications 1 à 5, dans lequel la racémisation est effectuée en combinaison avec un processus de résolution.

7. Processus selon la revendication 6, dans lequel la racémisation est effectuée en combinaison avec un processus de résolution enzymatique.

8. Processus selon la revendication 6, dans lequel la racémisation est effectuée en combinaison avec une résolution induite par cristallisation.

9. Processus selon l'une quelconque des revendications 6 à 8, dans lequel le processus de résolution est combiné à une racémisation *in situ.*

10. Processus selon la revendication 9, dans lequel la racémisation est effectuée *in situ* dans un processus de transformation asymétrique induite par cristallisation.
